# EUROPEAN PATENT SPECIFICATION

(11) **EP 4 496 543 B1**
(45) Date of publication and mention of the grant of the patent: **23.09.2026**
(21) Application number: 23717326.5
(22) Date of filing: 22.03.2023
(51) Int. Cl.: A61H 1/02

(54) **INTERFACE FOR AN EXOSKELETON**
SCHNITTSTELLE FÜR EIN EXOSKELETT
INTERFACE POUR UN EXOSQUELETTE

(30) Priority: 22.03.2022 US 202263322548 P
(43) Date of publication of application: 29.01.2025
(73) Proprietor: OSSUR ICELAND EHF, 110 Reykjavik (IS)
(72) Inventor: OMARSSON, Bjorn, 110 Reykjavik (IS); ALBANA, Sonia, 56025 Pontedera (PI) (IT); MORELLI, Luca, 56025 Pontedera (PI) (IT)
(74) Representative: Winger
(86) International application number: PCT/US2023/015866
(87) International publication number: WO 2023/183364

(56) References cited:
- EP-A1- 3 851 087
- WO-A1-2021/092041
- US-A1- 2014 018 715
- US-A1- 2018 303 699

## Description

### TECHNICAL FIELD

The embodiments of this disclosure relate to an interface for an exoskeleton arranged to reduce the physical effort of users.

### BACKGROUND

A stroke is a medical condition that affects the arteries leading to and within the brain. During a stroke, a patient's brain is deprived of oxygen and nutrients, resulting in damage to the brain. After a stroke, patients may experience a loss of normal function of part of their body, including the loss of movement and sensation such as experiencing weakness or paralysis on one side of their body; the loss of their ability to speak, eat, or swallow; the loss or decline of vision, cognitive ability, perception and orientation; the loss in their ability to maintain self-care; and the ability to control their emotions, and bowel and bladder movements.

These effects depend on which part of the brain is affected by the stroke. For instance, a right hemisphere stroke may cause patients experiencing the effects mentioned above on the left side of their body. A left hemisphere stroke may cause patients undergoing these effects on the right side of their body. Thus, stroke patients may be only impaired on one side of their body. These impairments may impede stroke patients from engaging in basic motor-function-related tasks such as walking, climbing stairs, starting, stopping, sitting, standing, dressing, feeding themselves, and implementing oral hygiene. These impairments lead to individually losing personal freedom and a reduction in their quality of life. Patients are limited in their mobility in daily life activities, participation in society, and the likelihood of returning to professional activities.

Exoskeletons are becoming valuable tools for addressing needs in healthcare and industrial applications. These exoskeletons can give a user improved endurance and stability or provide corrections to an impaired individual's motor functions by applying mechanical forces to the body in parallel with the user's muscles. These assistive and rehabilitative bionics technologies have the potential to improve quality of life, reduce the incidence of injury, and create a safer, more comfortable, and more productive environment.

An example of an exoskeleton for aiding stroke patients is an upper-body orthosis device which is a wearable exoskeleton arranged to offset gravitational forces acting on an impaired arm of the user as the user moves the arm. The upper-body orthosis device may be of the type described in WO 2012/099995A2 or US 2020/0139537, which are designed for vertical support of the arms of a user to assist him in tasks to be performed in positions where his arms are raised. Document US 2018303699 A1 shows an interface system in an exoskeleton, including a base support, a posterior strut, and a strap assembly that together anchor the exoskeleton to the wearer's body. The strap assembly includes shoulder/upper-torso straps that connect from transverse members of the posterior strut over the user's shoulders and anchor to the base support (e.g., a cinchable waist belt) in the front. The length and attachment of the straps are adjustable, allowing customization for different body sizes and distributing loads from assistive devices through the shoulders and waist, for securing the exoskeleton to the user's torso with comfortable, adjustable wearable straps.

Traditionally, upper-body or shoulder exoskeletons are bilateral but may be implemented in a unilateral configuration depending on the application. One such application may be for stroke patients experiencing asymmetric symptoms such as impairments on only one side of their body. In such circumstances, a unilateral shoulder exoskeleton is better suited for rehabilitating the impaired side while leaving the unimpaired side free from interference by the exoskeleton. However, the stability of interfaces supporting unilateral upper-body exoskeletons has proven problematic as such interfaces rotate toward the actuated side due to the asymmetric weight distribution and moments applied by the connected exoskeleton. These asymmetries often result in a misalignment between the user's anatomical and the exoskeleton's robotic joints. The resulting misalignments further result in the lower attachment points of the shoulder straps decoupling from their anchor positions, causing the interface for the upperbody exoskeleton to disengage from a user or cause discomfort and risk of injury.

There remains a need to develop an interface for a unilateral upper-body exoskeleton that will resist and prevent a connected unilateral upper-body exoskeleton from rotating toward the actuator.

Other known problems in interface design include discomfort resulting from pressure points caused by tightly connected components with a user's body, such as arm attachments causing pressure points on a user's arm or straps interfering with sensitive body parts such as shoulder straps in upper-body exoskeletons interfering with a user's breasts. Additionally, tightly or well-fit components are often challenging to access when donned by a user.

Lastly, interfaces for upper-body exoskeletons can be expensive to manufacture and for individuals to afford. While consumers have limited funds and resources and have varying sizes, comfort preferences, and tolerances, and may either gain or lose weight affecting their sizing, there is a need to develop a one-size-fits-all interface for upper-body exoskeletons capable of adapting to the user's specifications without causing pain or discomfort.

### SUMMARY

The disclosure describes various embodiments of an interface for use with an exoskeleton. The invention is set out in the appended set of claims.

The interface includes two shoulder straps configuration or an additional stability strap designed to counteract the moment of rotation caused by an asymmetrically connected upper-body exoskeleton. The shoulder straps may extend from the strut over a user's shoulders and secure to a belt fastened around the user's waist. The stability strap extends from the strut in a direction opposite that of the upper-body exoskeleton to an independently secured element.

The shoulder and stability straps may include length adjusters to accommodate the user's preferences and comfort while maintaining adequate tensile force to withstand the moment caused by the asymmetrically located upper-body exoskeleton.

The belt may be configured in a one-size-fits-all configuration having a plurality of attachment points to enable users to customize the fit of the interface for use with an upperbody exoskeleton.

The strut is in a T-shape having two arms extending from a central upright portion of the strut. The stability strap is arranged to extend from the T-arm opposite the shoulder kinematic chain downward along the user's back to a position on the belt located anterolaterally of the user. Preferably, the stability strap secures to the belt on the same side of the belt as the T-arm from which the stability strap extends.

These and other features, aspects, and advantages of the present disclosure will become better understood regarding the following description, appended claims, and accompanying drawings.

### GLOSSARY

"actuated arm" connotes the arm of a user that is fitted with a unilateral upper-body exoskeleton.
"actuated side" connotes the side of the interface that is connected to a unilateral upper-body exoskeleton corresponding to the actuated arm.
"anterolateral (or anterolaterally)" connotes in front and to the side describing a position around a human hip corresponding to a position laterally located from the groin near where the hip flexor muscles attach.
"bilateral exoskeleton" connotes an exoskeleton that assists two limbs.
"bilateral exoskeleton" connotes an exoskeleton that assists two limbs.
"comfort" connotes a state of physical ease and freedom from pain or constraint;
"comfortable fit" connotes fitting closely such that the component is flush against the user's body without gaps between the user and the respective components and without causing a state of physical unease.
"exoskeleton" connotes bionic components that assist, augment, or amplify a human user's movement in performing a physical function and may include electric motors, pneumatics, levers, hydraulics, or springs.
"first arm" connotes a portion of the strut extending from the central upright portion of the strut located on one side of the sagittal plane.
"interface or (physical Human-Robot interface ("pHRI"))" connotes components used to pair the exoskeleton with the human user by acting as an intermediate component between the human user and the exoskeleton providing support and comfort to the human user.
"interfere" connotes contact with or limiting the motion of a user's muscles or limbs, thereby affecting comfort.
"shoulder kinematic chain" refers to a mechanism adapted to assist users by replicating the physiological movements of the user and may incorporate features found in PCT/US2012/021770, filed on January 18, 2012, and published as WO 2012/099995A2 on July 26, 2012, and in US 2020/0139537, published May 7, 2020. The references are merely exemplary, and the shoulder kinematic chain may have any suitable construction, composition, or configuration.
"lateral (or laterally)" connotes away from the midline of the body defined by the sagittal plane.
"line of action" connotes the line through the point at which a force is applied in the same direction as a corresponding vector.
"medial (or medially)" connotes toward the midline of the body defined by the sagittal plane.
"moment" connotes the product of the magnitude of the force and the perpendicular distance between the line of action of the force and the pivot point.
"plurality" connotes two or more.
"posterolateral (or posterolaterally)" connotes behind and to the side and describes a position around a human hip corresponding to a position near the backside of the iliac crest.
"prolonged" connotes a period extending through the day corresponding to several hours.
"rigid" connotes void of flexibility sufficient to resist being forced out of shape.
"sagittal plane" connotes the longitudinal plane which divides the human body about a midline into right and left parts.
"second arm" connotes a portion of the strut extending from the central upright portion of the strut located on one side of the sagittal plane (the second arm being opposite the first arm).
"secured element" connotes a fixed or fastened component that is independent of the strut.
"slack" connotes loose or void of tension.
"stable" connotes firmly fixed and not likely to give way.
"taut" connotes stretched or pulled tight.
"tensile strength" connotes resistance of a material to breaking under tension.
"unactuated arm" connotes an arm of a user that is not fitted with a unilateral upper-body exoskeleton.
"unactuated side" connotes the side of the interface that is not connected to a unilateral upper-body exoskeleton and is opposite the actuated side.
"unilateral exoskeleton" connotes an exoskeleton that only assists one limb.
"well fitted" connotes fitting closely such that the component is flush against the user's body without gaps between the user and the respective components.

### DESCRIPTION OF THE DRAWINGS

Fig. 1 is a perspective view of an interface for use with a unilateral upper-body exoskeleton.
Fig. 2 is a rear view of an exemplary embodiment of an interface with a stability strap illustrating an exemplary moment and corresponding resistance force.
Fig. 3 is a side view of an exemplary embodiment of the stability strap engaging with a shoulder strap.
Fig. 4 is a side view of an exemplary embodiment of the stability strap engaging with a belt.
Fig. 5 is a rear view of the anatomy of a human back.

The figures are not necessarily drawn to scale but instead are drawn to understand the components better and are not intended to be limited in scope but to provide exemplary illustrations. The figures illustrate exemplary configurations of stable unilateral upper-body exoskeletons and in no way limit the structures or configurations of an upper-body exoskeleton system and components thereof, including stability straps according to the present disclosure.

### DETAILED DESCRIPTION

A better understanding of different embodiments of the disclosure may be had from the following description read with the accompanying drawings in which reference characters refer to like elements.

While the disclosure is susceptible to various modifications and alternative constructions, certain illustrative embodiments are in the drawings and are described below..

It will be understood that unless a term is expressly defined in this application to possess a described meaning, there is no intent to limit the meaning of such term, either explicitly or indirectly, beyond its plain or ordinary meaning.

A description of a few terms may be useful for further ease of understanding the embodiments of an upper-body exoskeleton. The embodiments of the upper-body exoskeleton may correspond to anterior and posterior body sections defined by an anterior-posterior plane and lateral body sections defined by a medial or sagittal plane. The anatomical terms described are not intended to detract from the standard understanding of such terms as readily understood by one of ordinary skill in the art of orthopedics, braces, human interfaces, and supports.

These anatomical terms follow the user wearing the upper-body exoskeleton, referring to an anatomical position. An anatomical position is generally defined as the erect position of the body with the face directed forward, the arms at the side, and the palms of the hands facing forward, which is a reference in describing the relation of body parts to one another.

The embodiments of the disclosure are adapted for a human body and may be dimensioned to accommodate different types, shapes, and sizes of human body sizes and contours. For explanatory purposes, the upper-body exoskeleton embodiments described are referred to as corresponding to other sections of a body and are denoted by general anatomical terms for the human body.

One of ordinary skill in the art will understand that this disclosure includes numerous other implementations. While some examples described may refer to functions performed by given actors such as "users" and/or other entities, it should be understood this description is for explanation only. The claims should not be interpreted to require action by any such exemplary actor unless explicitly required by the language of the claims themselves.

Generally, physical Human Robot interfaces ("pHRI") are used to pair an exoskeleton with a human user. These interfaces assist users by aligning the robotic joints of the exoskeleton with the anatomical joints of the human user while providing adequate support and comfort to enable a user to don the exoskeleton for a prolonged period. Typically, these interfaces include a rigid or semi-rigid frame defining attachment points from which an exoskeleton may be attached and from which straps may extend.

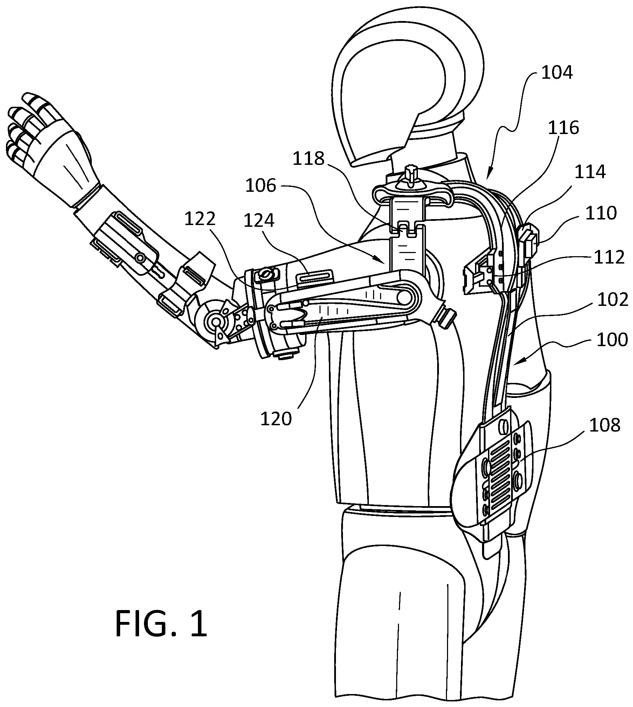

Fig. 1 shows an exemplary physical Human-Robot interface ("pHRI") 100 for securing an exemplary unilateral upper-body exoskeleton 106 to a human user. The exemplary pHRI 100 may include a strut 102 connected to a unilateral upper-body exoskeleton 106 via a shoulder kinematic chain 104, a lumbar support 108, and a plurality of straps (not shown in Fig. 1) to secure the pHRI 100 in place. The strut 102 may be a rigid component or frame configured to enhance a comfortable interaction between the user and the pHRI 100, provide back support to a user, balance and stabilize the unilateral upper-body exoskeleton 106 on a user's body, and safely transfer loads from the user's shoulders to the user's hips. The strut 102 may take various forms, including a T-shape with a first arm 110 and a second arm 112.

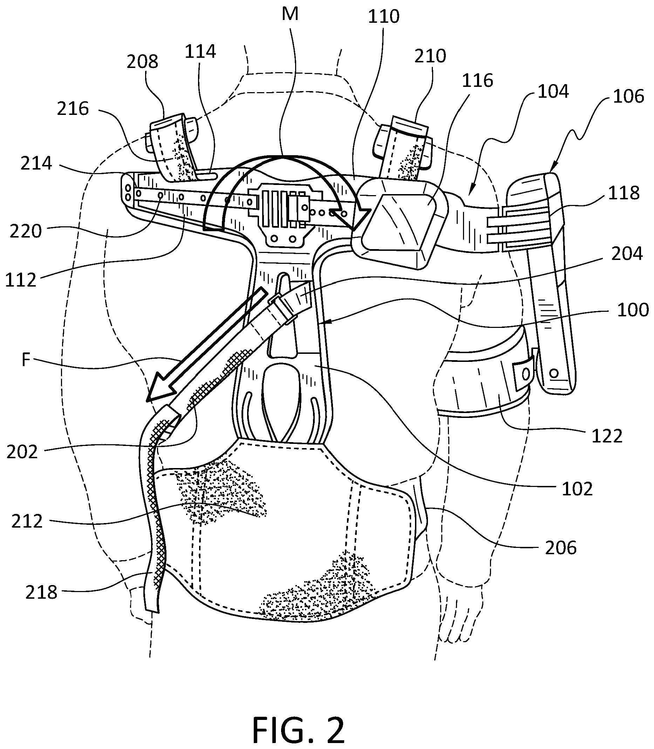

The first and second arms 110, 112 may extend from a central upright portion of the strut 102 to form a T-shape, as shown in Figs. 1 and 2. The T-shaped strut 102 may be desirable for adjusting the horizontal alignment between the unilateral upper-body exoskeleton 106 and the user's anatomy by providing laterally placed "arms" 110, 112. The position of the shoulder kinematic chain 104 may be laterally adjusted to accommodate the anatomy of the user. The strut 102 may be a substantially vertical beam such as a vertical bar shape or may be any other desirable shape providing for the attachment of a shoulder kinematic chain 104. The strut 102 may connect with a lumbar support 108 configured to support the user's lower back and may include one or more strap slots 114 configured to receive shoulder straps (not shown in Fig. 1).

The pHRI 100 may incorporate a garment or shoulder straps configured to wrap around the user's shoulders. The user may don the interface similar to a backpack. A belt 206 may also be implemented to secure the lower portion of the pHRI 100, including the lumbar support 108, around the user's waist.

The connected unilateral upper-body exoskeleton 106 extending from the shoulder kinematic chain 104 may assist users by replicating the physiological movements of the user and may incorporate features found in PCT/US2012/021770, filed on January 18, 2012, and published as WO 2012/099995A2 on July 26, 2012, and in US 2020/0139537, published May 7, 2020. The references are merely exemplary, and the unilateral upper-body exoskeleton 106 may have any suitable construction, composition, or configuration.

The shoulder kinematic chain 104 may extend from the strut 102, having a first end 116 and a second end 118 with one or more link and joint members therebetween. The first end 116 may connect to a position on the strut 102 corresponding to the user's shoulder width so the shoulder kinematic chain 104 is aligned with the user's shoulder joint. For instance, the first end 116 of the shoulder kinematic chain 104 may extend from the first arm 110 of the strut 102, as shown in Fig. 1. The shoulder kinematic chain 104 may be further configured to be aligned around the user's shoulder joint to increase the shoulder's range of motion and guarantee freedom of movement. For example, the torque generating device 120 may be connected to the second end 118 of the shoulder kinematic chain 104.

The torque generating device 120 is a component configured to provide a torque upon the user. The torque generating device 120 may contain an elastic mechanical component configured to apply a torque on the user corresponding to the angle of deflection, similar to the device disclosed in US 2020/0139537 A1. The torque generating device 120 may include an electric motor or rotary spring to provide torque to the user's arm. The torque generating device 120 may contain the torque producing elements within a housing having a medial side and a lateral side, wherein the torque generating device 120 is located laterally of a user's arm when donned so the medial side of the torque generating device 120 is adjacent to and runs parallel with the user's arm.

The torque generating device 120 may further include an arm attachment component 122 extending from the medial side of the torque generating device 120 configured to engage with the arm of the user so the mechanical forces are adequately transferred from the unilateral upper-body exoskeleton 106 to the user. The arm attachment component 122 may include a D-ring 124 configured to receive a strap or cuff of sufficient length to wrap around a user's arm for securing a user to the torque generating device 120. For instance, the cuff may exceed 60 centimeters to accommodate individuals with large arms, such as bodybuilders or severely overweight individuals. The cuff may accommodate average arm sizes. The cuff may include hook-and-loop fastening elements so the cuff may be inserted through the opening defined by the D-ring 124 and wrapped around to be secured to itself.
The D-ring 124 may be configured to bend away from the user's arm or laterally toward the lateral surface of the torque generating device 120. When so configured, the D-ring 124 does not interfere with the user's arm, providing greater access to the D-ring 124 and preventing pressure points and discomfort experienced by the user.

The connected unilateral upper-body exoskeleton 106 and shoulder kinematic chain 104 may be sufficiently heavy to cause the strut 102 to rotate about the base of the strut 102. As shown in Fig. 2, the shoulder kinematic chain 104 is connected to the first arm 110 of the T-shape strut 102. The weight and location of where the shoulder kinematic chain 104 attaches to the strut 102 at the first end 116 create a moment M causing the strut 102 to rotate. The rotation of the pHRI 100 may cause misalignments between the pHRI 100, unilateral upper-body exoskeleton 106, and the anatomy of the user.

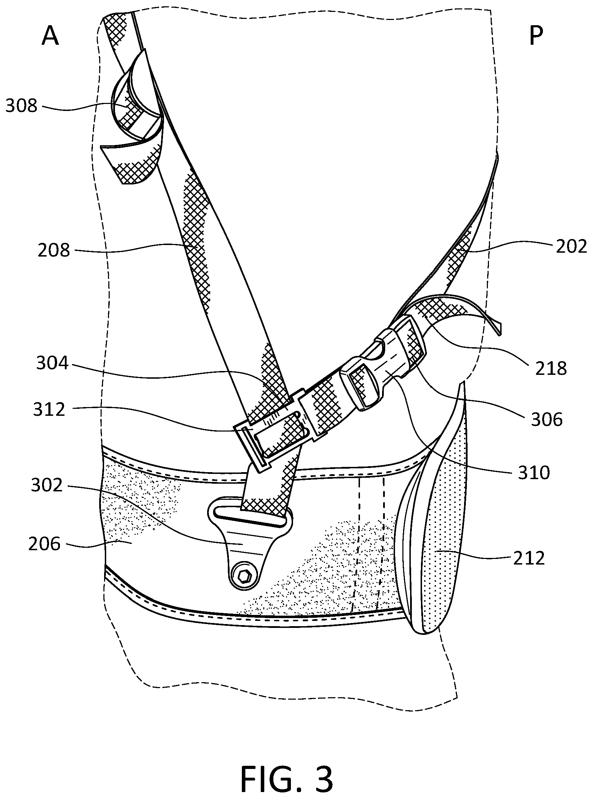

Additionally, this misalignment disconnects the shoulder straps from the lower portion of the strut 102 when shoulder straps 208, 210 are implemented to extend from the strut 102, around a user's shoulders, and connect again the strut 102. This arrangement may cause the pHRI 100 to disengage from a user, causing more significant discomfort or risk of injury upon the user. This misalignment may be overcome by anchoring the first and second shoulder straps 208, 210 to the belt 206 and/or providing a stability strap 202, exerting a force F to counteract the moment M applied by the shoulder kinematic chain 104, as shown in Fig. 3.

The following discussion relates to Figs. 2-4. Fig. 2 shows an exemplary pHRI 100 having a stability strap 202, a belt 206, a first shoulder strap 210 connected to the actuated first arm 110 of the strut 102, and a second shoulder strap 208 connected to the non-actuated second arm 112 of the strut 102. Although Fig. 2 shows the user's right arm as the actuated arm and the user's left arm as the unactuated arm, other configurations are possible where the user's left arm is the actuated arm, and the user's right arm is the unactuated arm. The first arm 110 corresponds with the actuated side and can be on either the right or left side, and the second arm 112 corresponds with the unactuated side and can be on either the right or left side.

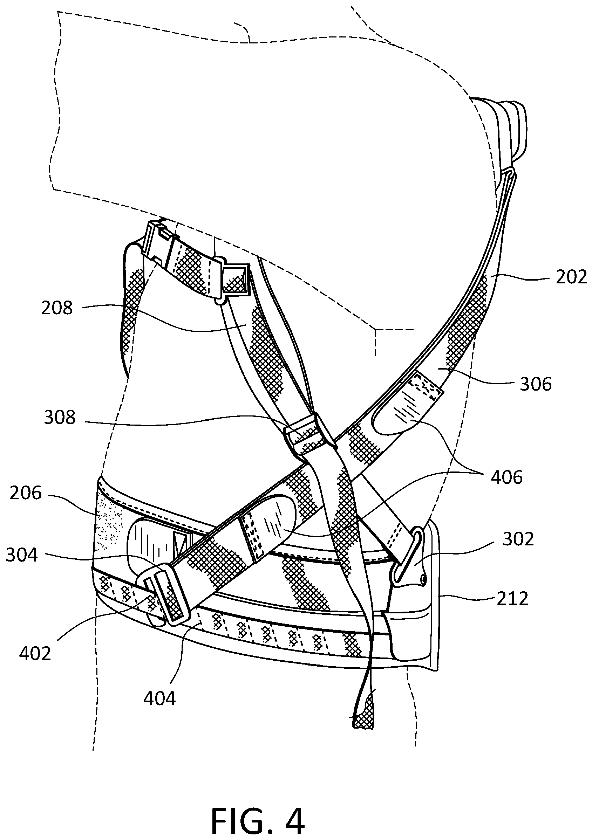

Fig. 3 shows an exemplary embodiment of a pHRI 100 with a second shoulder strap 208 anchored to a belt 206 and a stability strap 202 securing to the second shoulder strap 208. Fig. 4 shows an exemplary embodiment of a pHRI 100 having a second shoulder strap 208 anchored to a belt 206 at a posterolaterally located position and a stability strap 202 securing to the belt 206 at an anterolaterally located position on the same side of the sagittal plane as the second shoulder strap 208.

The stability strap 202 may include an upper connector 204 configured to engage with the strut 102 and a lower connector 304 configured to engage with a secured element. The secured element is preferably independent of the strut 102 for the stability strap 202 to resist the strut 102 better since a dependent element may pivot with the strut 102, resulting in the stability strap 202 providing less resistance to the movement of the strut 102. The secured element may be the belt 206, the second shoulder strap 208, or another stable object that is independent of the motion of the strut 102.

The stability strap 102 may extend laterally from the strut 102 in the direction opposite the shoulder kinematic chain 104. The stability strap 102 connects to the secured element on the opposite side of the sagittal plane as the shoulder kinematic chain 104. In this configuration, the stability strap 202 will resist movement caused by the weight and location of the shoulder kinematic chain 104 and maintain the position of the strut 102. If the shoulder kinematic chain 104 is located on the right side of the strut 102, such an arrangement will cause the strut to rotate towards the right. Consequently, the stability strap 202 may extend from the strut 102 to the left and engage with a point on a secured element so the stability strap 202 counteracts the torque caused by the shoulder kinematic chain 104.

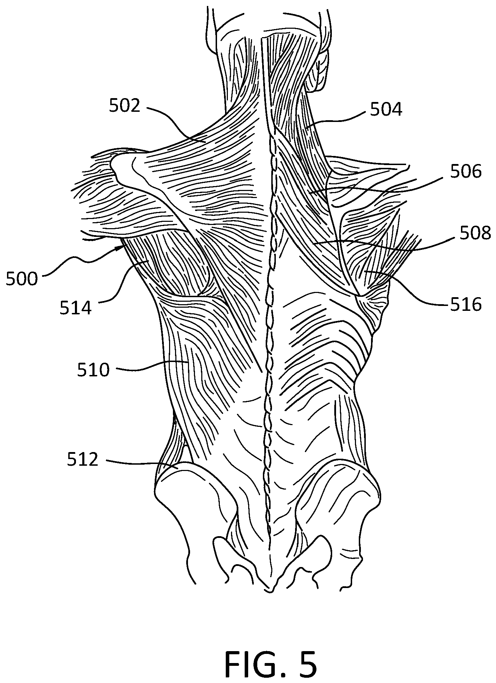

The configuration of the stability strap 202 may be chosen based on balancing factors, including maximizing the resistance force F of the stability strap 202 and reducing discomfort to a user's armpit 500, trapezius 502, levator scapulae 504, rhomboid minor 506, rhomboid major 508, or latissimus dorsi muscles 510, shown in Fig. 5.

The first factor is an objective determination of ensuring the stability strap 202 applies and can withstand the force F required to counteract the moment M induced by the weight and location of the shoulder kinematic chain 104. Because a moment is the product of the magnitude of the force and the perpendicular distance between the line of action of the force and the pivot point, the stability strap 202 can be configured anywhere along the strut 102 if the stability strap 202 has a tensile strength sufficient to withstand the force F required to counteract the moment M caused by the shoulder kinematic chain 104. For example, for a given moment M, the force F needed to counteract the moment M will differ depending on how far the applied force F is from the pivot point.

A force F applied along the central upright portion of the strut, as shown in Fig. 2, will be greater than a force F applied along the second arm 112 to counteract the moment M. Accordingly, although a stability strap 202 located along the second arm 112 of the strut 102 would require less force to counteract the rotation of the strut 102 than a stability strap 202 located near the base of the strut 102, the counteracting moment caused by the stability strap 202 upon the pivot point is the same because the distances from the pivot points make up for the reduced force.

Most materials will withstand the requisite resistance force F needed to counteract the moment M caused by shoulder kinematic chain 104 regardless of where the stability strap 202 is located. As a result, stability straps 202 can be made from various fabrics and materials, including nylon and polyester.

The second factor is a subjective determination for reducing discomfort experienced by a user. For example, a first user may find it more comfortable for the stability strap 202 to cross over their latissimus dorsi muscles 510 than to interfere with their armpit 500 or teres major muscles 514 while a second user may find it more comfortable for the stability strap 202 to cross over another area of their back. Accordingly, the stability strap 202 may be configured to extend from a plurality of locations along the strut 102 to the secured element.

In a first exemplary embodiment, as shown in Fig. 3, the first and second shoulder straps 208, 210 extend from the strap slots 114 and anchor to the belt 206 at positions located laterally along the belt 206. The stability strap 202 extends from the central upright portion of the strut 102 to a position on the second shoulder strap 208 near the anchor point of the second shoulder strap 208. The stability strap 202 may be secured anywhere along the central upright portion of the strut 102. In this first exemplary embodiment, the stability strap 202 crosses over the user's latissimus dorsi muscles 510 to avoid the user's armpit 500, trapezius 502, or rhomboid muscles 506, 508. Preferably, the lower connector 304 of the stability strap 202 may be secured at a position along the second shoulder strap 208 that is as close to the belt 206 as possible to maximize comfort in this configuration.

In a second exemplary embodiment, as shown in Fig. 4, the stability strap 202 extends from the second arm 112 of the strut 102 to the belt 206 at a position located anterolaterally on the same side of the sagittal plane as the second arm 112 of the strut 102. The stability strap 202 may connect to the strut 102 anywhere along the attachment elements 214 of the second arm 112. The stability strap 102 experiences less tensile force F to resist the moment M caused by the shoulder kinematic chain 104, the further the stability strap 102 is laterally connected from the central upright portion of the strut 102. In this second exemplary embodiment, the stability strap 202 crosses over the user's trapezius 502, teres major 514 and infraspinatus muscles 516, and avoids most of the latissimus dorsi muscle 510. Other configurations are also possible, including combinations of the above-mentioned exemplary embodiments.

Preferably, the upper connector 204 of the stability strap 202 is anchored to the second arm 112 on the strut 102 instead of the central upright portion because the stability strap 202 will provide the requisite force F to counteract the moment M caused by the shoulder kinematic chain 104 while more comfortably interfering with the armpit 500, teres major 514 and infraspinatus muscles 516 than the latissimus dorsi muscles 510 of the lower back.

The upper connector 204 may be permanently secured to the strut 102 using a rivet or temporarily secured to the strut 102 to enable future adjustments to the stability strap 202. A plurality of mechanical fastening elements may be permanently attached to the strut 102 to provide a plurality of potential attachment positions for the stability strap 202, including a plurality of D-rings or a plurality of mounting holes 220 defined by the strut 202, as shown in Fig. 2, configured to receive either a quick-detach connector like the one disclosed in US 8,832,986 B2, a hook, or other insertable fastening devices. Variations may include a linear guide having mounting features configured to connect to the stability strap 202. The linear guide mounted on a linear slide is configured to move laterally and medially and be secured along the second arm 112 of the strut 102.

The lower connector 304 may be any device capable of securing the stability strap 202 to the secured element and may be chosen based on how the stability strap 202 is implemented. For instance, in the first exemplary embodiment, the lower connector 304 is a slidable strap buckle configured to slide along the second shoulder strap 208, facilitating the height adjustment of the stability strap 202 and corresponding fit. In the second exemplary embodiment, the lower connector 304 is a hookable clasp configured to engage with fastening mechanisms on the belt 206. A variation for coupling the stability strap 202 to the secured element involves using a mushroom-head shaped element or similarly shaped element configured to engage with the secured element by keyholes.

Proper alignment of the pHRI maintains a comfortable fit for the user. Individuals have various sizes and comfort tolerances. The same size stability strap 202 used with two users may provide varying degrees of comfort. For instance, a first user may be larger than a second user. The first user may experience a taut stability strap 202 and may find it uncomfortable, while the second user may experience a looser stability strap 202. If the pHRI 100 is uncomfortable, users may not be inclined to use the device for a prolonged period, thereby diminishing the benefits of the connected unilateral upper-body exoskeleton 106 and impeding their recovery.

Due to the diversity in body types that may use the pHRI 100 and connected unilateral upper-body exoskeleton 106, means can be provided to adequately counter the moment M caused by the shoulder kinematic chain 104 for all potential users. The same size stability strap 202 used with two users may provide differing counteracting forces F resulting in varying degrees of movement along the strut 102 depending on how taut the stability strap 202 is implemented. For instance, a first user may be larger than a second user. The first user may benefit from a taut stability strap 202. The second user may experience a stability strap 202 with slack which may not adequately provide sufficient tension to counteract the moment M caused by the shoulder kinematic chain 104. If the pHRI 100 rotates, causing the unilateral upper-body exoskeleton 106 to become misaligned with the user's anatomy, the device may injure the user. Accordingly, the pHRI 100 is preferably adjustable to conform to the user's size and comfort preferences.

The stability strap 202 may be adjustable to adapt to a user's size and comfort preferences by implementing a stability strap adjuster 306. In a first exemplary embodiment, the stability strap 202 may be both vertically and horizontally adjustable by selecting the lower connector 304 of the stability strap 202 as a slidable strap buckle 312 connected to a stability strap adjuster 306, as shown in Fig. 3. In addition, the slidable strap buckle 312 may be slidably connected to the second shoulder strap 208 so the stability strap 202 is slidable along the second shoulder strap 208. The stability strap adjuster 306 may be a non-slidable strap buckle configured to receive the stability strap 202. The non-slidable strap buckle may be of the type described in US 4,502,191 or US 4,171,555.

The stability strap 202 may be connected to the lower connector 304 by feeding the stability strap 202 through a first opening of the non-slidable strap buckle, wrapping the stability strap 202 around a central rod portion of the non-slidable strap buckle, and feeding the stability strap 202 through a second opening of the non-slidable strap buckle leaving an end portion 218 extending from the buckle strap. In such embodiments, the effective length of the stability strap 202 may be shortened by pulling on the end portion 218 until the stability strap 202 is properly tightened. Likewise, the effective length of the stability strap 202 may be lengthened by reverse feeding the stability strap 202 through the strap buckle.

In a second exemplary embodiment, the stability strap adjuster 306 may comprise hook-and-loop features located along one side of the stability strap 202 so the hook features engage with the loop features when the stability strap 202 is folded around a bracket, as shown in Fig. 4. The brackets may be any device capable of securing the stability strap 202, such as D-rings or strap buckles. The brackets may be the upper and lower connectors 204, 304 and located at the above-mentioned anchoring points of the stability strap 202.

In such embodiments, the stability strap 202 having hook-and-loop features located along the length of one side of the stability strap 202 may be inserted through the brackets such that the ends of the stability strap 202 extending through the brackets may fold over and fasten to itself. For the stability strap 202 to secure itself, the side of the stability strap 202 having the hook-and-loop features may be located along the interior side. The hook-and-loop features are sandwiched between the two segments of the stability strap 202 when secured to itself.

The effective length of the stability strap 202 may be shortened by pulling the ends 406 of the stability strap 202 through the buckles and securing the ends 406 centrally along the stability strap 202. Conversely, the effective length of the stability strap 202 may be lengthened by reverse feeding the stability strap 202 through the buckles and securing the ends 406 to an outer position along the stability strap 202. The adjustability of the stability strap 202 allows the user to adjust the length of the stability strap 202 to enhance the comfort and functionality of the pHRI 100.

Additionally, the stability strap 202 may include other features to facilitate quick donning and doffing of the pHRI 100. For instance, an exemplary stability strap 202 may have a quick release element 310, as shown in Fig. 3. The quick-release element 310 may enable clinicians, social workers, and others to assist better users donning and doffing the pHRI 100. The quick-release element 310 may be more applicable in embodiments where the lower connector 304 of the stability strap 202 is permanently or semi-permanently connected to a secure element so the stability strap 202 is not readily detached as shown in Fig. 3. In embodiments where the lower connector 304 may be more readily detached from the secured element, as shown in Fig. 4 with the hookable clasp, a quick-release element 310 may not be needed.

The quick-release element 310 may be any mechanical device capable of quickly securing two components together, such as snap buckles shown in Fig. 3 or described in US 7,895,719 B2 and US 5,991,985 A. Alternatively, the quick release element 310 may include hook-and-loop features, zippers, buttons, or snap buttons.

The stability strap 202, when donned, may be tightened until well-fitted so the stability strap 202 fits closely and comfortably to the user. The stability strap 202 is sufficiently taut when the experienced tensile force F of the stability strap 202 is at least equal to the weight of the shoulder kinematic chain 104 multiplied by the ratio between the perpendicular distances of the shoulder kinematic chain 104 and the stability strap 202 from the pivot point, respectively. For instance, if a 1.5 kg shoulder kinematic chain 104 is located on the first arm 110 positioned 0.1 meters from the central upright portion of the strut 102, the shoulder kinematic chain 104 applies a moment of 1.47 Nm on the pivot point. A stability strap 202 located on the second arm 112 may be positioned 0.2 meters from the central upright portion of the strut 102 will need to be tightened until having a tensile force of 7.35 N. If the requisite tautness of the stability strap 202 is uncomfortable for the user, then the location of where the stability strap 202 is connected to the strut 102 may be moved laterally to require a reduced force F.

The belt 206 engages with the strut 102, secures the lower portion of the pHRI 100 against a user's body via a lumbar pad 212, and serves as an anchor for the first and second shoulder straps 208, 210, and the stability strap 202. The belt 206 may be well fitted and stable to adequately transfer forces from the pHRI 100 to the user's waist and prevent the pHRI 100 from becoming loose or moving along with the rotation of the strut 102, thereby becoming dependent upon the strut 102. The belt 206 may be breathable to keep the skin cool and comfortable and may have additional features to ease the donning and doffing of the belt 206 by users. For instance, the belt 206 may be a Miami LSO style belt.

The belt 206 is sufficiently long to wrap around a user's waist and may be configured to rest along the top of the user's iliac crest 512, as shown in Figs. 2-5. Due to the diversity in body types, the belt 206 may be designed in various ways to accommodate the diversity of users. For instance, the belt 206 may be a single band with a length exceeding the circumference of the user's waist or hips, or the belt 206 may be adjustable, having one or more hip-belt straps marked with indicia of measurement as disclosed in US 9,462,875 B2. Alternatively, the belt 206 may be designed in other one-size-fits-all configurations.

A one-size-fits-all configuration is preferred for a few reasons, including reducing costs for manufacturers and consumers as manufacturers can reduce costs by producing a single design for a belt 206 that can be used for all consumers, and consumers can purchase a single belt 206 without worrying about fluctuations in their body size. Additionally, while a user or patient may purchase a sized device, it may be too cumbersome for a rehabilitation center to carry multiple sizes. A one-size-fits-all configuration is also beneficial in rehabilitation settings where the pHRI 100 may be fitted to multiple patients during the day. The one-size-fits-all configuration may be more pertinent for electronically actuated embodiments of connected unilateral upper-body exoskeletons 106, which are likely to be more expensive than spring-actuated embodiments.

However, a single designated mounting position for the first and second shoulder straps 208, 210 or the stability strap 202 may provide sizing issues and inadequate support for some users. For instance, a one-size-fits-all belt 206 having designated mounting positions may provide differing anchoring positions for different users resulting in varying degrees of comfort and assistance. For instance, a first user may be larger than a second user. The designated anchoring positions on the belt 206 may be located more posterior when donned by the first user while located more anterior when donned by the second user. Additionally, the anchoring position may be covered by the front closure of the belt 206 if the user is too small.

The one-size-fits-all belt 206 may resolve these issues by including a plurality of fastening locations to enable the stability strap 202 to engage with the belt 206 at locations appropriate for the user. In an exemplary embodiment, the plurality of fastening locations may include a series of slots 402 configured to receive a lower connector 304 such as a hookable clasp, as shown in Fig. 4. The slots 402 are separated by stitches 404 sufficiently spaced apart so each slot 402 can receive the lower connector 304. The plurality of fastening locations may include a plurality of keyholes configured to receive a mushroom-head or similar mechanical fasteners on the stability strap 202. The plurality of attachment points allows the coupling location of the lower connector 304 on the belt 206 to accommodate the entire range of user sizes.

In such embodiments, the user's body size is irrelevant. The user may secure the stability strap 202 to the locations positioned along the belt 206 that provides adequate force F to counteract the moment M caused by the shoulder kinematic chain 104 without causing discomfort.

Similarly, the one-size-fits-all belt 206 may include a plurality of fastening locations to enable the first and second shoulder straps 208, 210 to engage with the belt 206 at locations appropriate for the user. In some exemplary embodiments, a plurality of slots, keyholes, or D-rings may be implemented for the first and second shoulder straps 208, 210. In such embodiments, the user's body size is irrelevant as the user may anchor the first and second shoulder straps 208, 210 at a position adapted for their use.

The belt 206 may be fastened together through any means known in the art, including hook-and-loop fasteners, buckles, and laces.

The belt 206, when donned, may be tightened until well-fitted so the belt 206 fits closely and comfortably to the user. The belt 206 is sufficiently taut when the belt 206 rests flush against the user's body without gaps between the user and the belt 206, and at least part of the weight of the interface is transferred from the user's shoulders to the user's hips. The belt 206 may be provided with adequate padding so pressure points are not created when the belt 206 is securely fastened. If the requisite tautness of the belt 206 is uncomfortable for the user, then additional padding may be provided to disperse pressure points.

When donned, the first and second shoulder straps 208, 210 are configured to secure the upper portion of the pHRI 100 to the user's back. The first and second shoulder straps 208, 210 may have an upper attachment point 216 configured to engage with the strap slots 114 defined by the strut 102 and a lower attachment point 302 configured to engage with the belt 206 or the strut 102 at a position located below the strap slots 114. Preferably, the shoulder straps 208, 210 extend from the strap slots 114 and anchor to a position on the belt 206 to prevent decoupling due to misalignments caused by the shoulder kinematic chain 104.

For instance, the first shoulder strap 210 may be connected to the strap slot 114 on the first arm 110, and the second shoulder strap 208 may be connected to the strap slot 114 on the second arm 112. The first and second shoulder straps 208, 210 may extend from their respective strap slots 114 on the strut 102 to positions on the belt 206 so a user may don the pHRI 100 similarly as a backpack, where the first and second shoulder straps 208, 210 extend over the user's shoulders, downward along the user's torso to an anchoring point on the belt 206.

The lower attachment point 302 is susceptible to decoupling from the lower portion of the strut 202 during misalignment because the lower attachment point 302 is not independent of the movement of the strut 102 in this configuration. However, the belt 206 is independent of the strut 102 so the belt 206 remains stationary even if the strut 102 rotates due to the shoulder kinematic chain 104. The pHRI 100 is more securely donned when the shoulder straps 208, 210 are anchored to the belt 206.

In some exemplary embodiments the configuration of the first and second shoulder straps 208, 210 may be based on a gender-neutral design configured to avoid interfering with a user's breasts. In such exemplary embodiments, the first shoulder strap 210 may connect to the belt 206 on the same side of the sagittal plane as first arm 110 and the second shoulder strap 208 may connect to the belt 206 on the same side of the sagittal plane as the second arm 112. The first and second straps 208, 210 extend from their respective strap slots 114 downward along the user's torso to an anchor point on the belt 206. The first and second shoulder straps 208, 210 may also be anchored to the belt 206 at a position located more posterior to pull the first and second shoulder straps 208, 210 away from the user's chest. In this configuration, the straps avoid the chest area and the front of the body of the user.

The first and second shoulder straps 208, 210 may include shoulder strap adjusters 308 to facilitate tightening of the first and second shoulder straps 208, 210. The shoulder strap adjusters 308 may be any device known in the art to tighten straps including ratches or strap buckles.

The first and second shoulder straps 208, 210, when donned, may be tightened until well-fitted so the upper portion of the pHRI 100 fits closely and comfortably to the user. The first and second shoulder straps 208, 210 are sufficiently taut when the first and second shoulder straps 208, 210 rest flush against the user's torso and the upper portion of the pHRI 100 rests flush against the user's upper back without gaps between the user and the respective components. If the tautness of the stability strap 202 is uncomfortable for the user, then additional padding may be provided to relieve pressure points. The second shoulder strap 208 need not be as taut as the first shoulder strap 210.

The pHRI 100 may be donned by inserting the user's arms through the shoulder straps 208, 210, arranging the belt 206 around the user's waist or iliac crest 512, and coupling the stability strap 202 between the strut 102 and the secured element. The first and second shoulder straps 208, 210, the belt 206, and the stability strap 202 may be tightened or adjusted as discussed above. Preferably the belt 206 is tightened first, creating a solid foundation upon which the pHRI 100 may rest. Next, the first and second shoulder straps 208, 210 may be tightened, securing the pHRI 100. The stability strap 202 is preferably connected between the strut 102 and the secured element after the belt 206 and the first and second shoulder straps 208, 210 are tightened, so the position of the stability strap 202 is optimized to counteract the moment M caused by the shoulder kinematic chain 104. Finally, the stability strap 202 is tightened after the stability strap 202 is properly connected to the strut 102 and the secured element until it is sufficiently taut to counteract the moment M. This preferred order of operations ensures maximal stability by tightening the foundational elements first and the stability strap 202 last.

It should be understood that not necessarily all objects or advantages may be achieved under any embodiment of the disclosure. Those skilled in the art will recognize that the embodiments may be embodied or carried out to achieve or optimize one advantage or group of advantages as taught without achieving other objects or advantages as taught or suggested.

Those skilled in the art will recognize the interchangeability of various disclosed features.

While interface and corresponding exoskeleton are briefly described, they are not limited to the depicted embodiments and the interface system may be adapted to accommodate different shoulder assist mechanisms.

## Claims

1. A physical human-robot interface (100) for use with a upper body exoskeleton, the physical human-robot interface comprising: a rigid strut (102) defining one or more strap slots (114), the strut (102) adapted to correspond to a user's back; a shoulder kinematic chain (104) extending from the strut; a belt (206) connected to the strut (102); two shoulder straps (208, 210) configured to wrap around a user's shoulder and extending from the one or more strap slots (114) to the belt (206); ,
wherein the strut (102) is configured in a T-shape having a first arm (110) and a second arm (112), the first arm (110) and the second arm (112) extending from a central upright portion of the strut (102), wherein the shoulder kinematic chain connects to the first arm (110) at one side of the sagittal plane of the human-robot interface,
wherein the shoulder straps (208, 210) are connected to the first arm (110) and second arm (112) respectively, wherein the belt (206) is connected with the strut (102) along a base of the strut (102),
wherein one shoulder strap (208) anchors to the belt at a position posterolaterally or laterally located along the belt at the opposite side of the saggital plane as the shoulder kinematic chain (104),
wherein the interface further comprising a stability strap (202), **characterized in that** the stability strap (202) comprises a first end secured to a stability strap lower connector (304) engaging the one shoulder strap (208) that is anchored to the belt (206) at the opposite side of the saggital plane as the shoulder kinematic chain (104) or engaging the belt (206) at an anterolaterally located position on the same side of the sagittal plane as the one shoulder strap (208) anchored to the belt at the opposite side of the saggital plane as the shoulder kinematic chain (104), and
the stability strap (202) having a second end secured to a stability strap upper connector (204) engaging the strut from the central upright portion of the strut (102) or from the second arm (112) of the strut (102) such that the stability strap extends from the strut in a direction to the belt or the one shoulder strap (208) to which the stability strap is connected to a secure element, so as to counteract a moment of rotation caused by the shoulder kinematic chain (104).

2. The physical human-robot interface (100) of claim 1, wherein the one or more shoulder straps (208, 210) have an upper attachment point (216) and a lower attachment point (302), the one or more shoulder straps (208, 210) extending from the one or more strap slots (114) at the upper attachment point (216) to the belt (206) at the lower attachment point (302).

3. The physical human-robot interface (100) of claim 1, wherein the strut (102) further comprises a lumbar support (108).

4. The physical human-robot interface (100) of claim 3, wherein the belt (206) is connected to the lumbar support (108) of the strut (102).

5. The physical human-robot interface (100) of claim 4, wherein the belt (206) further comprises a lumbar pad (212).

6. The physical human-robot interface (100) of claim 1, wherein the first arm (110) and the second arm (112) define the one or more strap slots (114).

7. The physical human-robot interface (100) of claim 1, wherein the one or more shoulder straps (208, 210) have an upper attachment point (216) and a lower attachment point (302), the one or more shoulder straps (208, 210) extending from the one or more strap slots (114) at the upper attachment point (216) to the belt (206) at the lower attachment point (302); wherein the lower attachment points (302) connect laterally on the belt (206), or posterolaterally on the belt (206).

8. The physical human-robot interface (100) of claim 1, wherein the stability strap upper connector (204) connects to the second arm (112) and the stability strap lower connector (304) connects to the second shoulder strap (208).

9. The physical human-robot interface (100) of claim 1, wherein the stability strap upper connector (204) connects to the second arm (112) and the stability strap lower connector (304) connects laterally on the belt (206).

10. The physical human-robot interface (100) of claim 1, wherein the stability strap (202) connects to the belt (206) on the side corresponding to the second arm (112); wherein the stability strap (202) extends from the central upright portion of the strut (102) to a lateral position of the belt (206) corresponding to the side of the second arm (112).

11. The physical human-robot interface (100) of claim 1, wherein the stability strap (202) extends from a base of the central upright portion of the strut (102) to a lateral position of the belt (206) corresponding to the side of the second arm (112); wherein the stability strap (202) is configured to extend substantially horizontally around the latissimus dorsi muscles of a user to an anchoring point on the belt (206) located laterally of a user's hip.

12. The physical human-robot interface (100) of claim 1, wherein the stability strap (202) further comprises a stability strap quick release (310).

## Patentansprüche

1. Eine physische Mensch-Roboter-Schnittstelle (100) zur Verwendung mit einem Oberkörper-Exoskelett, wobei die physische Mensch-Roboter-Schnittstelle umfasst: eine starre Strebe (102), die einen oder mehrere Gurtschlitze (114) definiert, wobei die Strebe (102) angepasst ist, um dem Rücken eines Benutzers zu entsprechen;
- eine Schulterkinematikkette (104), die sich aus der Strebe erstreckt; einen Gürtel (206), der mit der Strebe (102) verbunden ist; zwei Schultergurte (208, 210), die konfiguriert sind, um sich um die Schulter eines Benutzers zu wickeln und sich von dem einen oder den mehreren Gurtschlitzen (114) zum Gürtel (206) erstrecken;
- wobei die Strebe (102) in T-Form konfiguriert ist, die einen ersten Arm (110) und einen zweiten Arm (112) aufweist, wobei sich der erste Arm (110) und der zweite Arm (112) von einem zentralen aufrechten Abschnitt der Strebe (102) erstrecken, wobei die Schulterkinematikkette an einer Seite der Sagittalebene der Mensch-Roboter-Schnittstelle mit dem ersten Arm (110) verbunden ist,
- wobei die Schultergurte (208, 210) jeweils mit dem ersten Arm (110) und dem zweiten Arm (112) verbunden sind, wobei der Gürtel (206) entlang einer Basis der Strebe (102) mit der Strebe (102) verbunden ist,
- wobei ein Schultergurt (208) an einer posterolateralen oder lateralen Position am Gürtel verankert ist, die sich entlang des Gürtels auf der gegenüberliegenden Seite der Sagittalebene der Schulterkinematikkette (104) befindet,
- wobei die Schnittstelle weiter einen Stabilitätsgurt (202) umfasst, **dadurch gekennzeichnet, dass** der Stabilitätsgurt (202) ein erstes Ende umfasst, das an einem unteren Verbinder (304) eines Stabilitätsgurts gesichert ist, der mit dem einen Schultergurt (208) in Eingriff steht, der auf der gegenüberliegenden Seite der Sagittalebene als Schulterkinematikkette (104) am Gürtel (206) verankert ist, oder der mit dem Gürtel (206) an einer anterolateral gelegenen Position auf derselben Seite der Sagittalebene wie der eine Schultergurt (208) in Eingriff steht, der auf der gegenüberliegenden Seite der Sagittalebene als Schulterkinematikkette (104) am Gürtel verankert ist, und
- der Stabilitätsgurt (202), der ein zweites Ende aufweist, das an einem oberen Verbinder des Stabilitätsgurts (204) gesichert ist, der vom mittleren aufrechten Abschnitt der Strebe (102) oder vom zweiten Arm (112) der Strebe (102) mit der Strebe in Eingriff steht, sodass sich der Stabilitätsgurt von der Strebe in Richtung des Gürtels oder des einen Schultergurtes (208) erstreckt, an dem der Stabilitätsgurt mit einem festen Element verbunden ist, um einem durch die Schulterkinematikkette (104) verursachten Drehmoment entgegenzuwirken.

2. Die physische Mensch-Roboter-Schnittstelle (100) nach Anspruch 1, wobei der eine oder mehrere Schultergurte (208, 210) einen oberen Befestigungspunkt (216) und einen unteren Befestigungspunkt (302) aufweisen, wobei sich der eine oder mehrere Schultergurte (208, 210) von dem einen oder mehreren Gurtschlitzen (114) am oberen Befestigungspunkt (216) zum Gürtel (206) am unteren Befestigungspunkt (302) erstrecken.

3. Die physische Mensch-Roboter-Schnittstelle (100) nach Anspruch 1, wobei die Strebe (102) weiter eine Lendenwirbelstütze (108) umfasst.

4. Die physische Mensch-Roboter-Schnittstelle (100) nach Anspruch 3, wobei der Gürtel (206) mit der Lendenwirbelstütze (108) der Strebe (102) verbunden ist.

5. Die physische Mensch-Roboter-Schnittstelle (100) nach Anspruch 4, wobei der Gürtel (206) weiter ein Lendenwirbelkissen (212) umfasst.

6. Die physische Mensch-Roboter-Schnittstelle (100) nach Anspruch 1, wobei der erste Arm (110) und der zweite Arm (112) einen oder mehrere Gurtschlitze (114) definieren.

7. Die physische Mensch-Roboter-Schnittstelle (100) nach Anspruch 1, wobei der eine oder mehrere Schultergurte (208, 210) einen oberen Befestigungspunkt (216) und einen unteren Befestigungspunkt (302) aufweisen, wobei sich der eine oder mehrere Schultergurte (208, 210) von dem einen oder mehreren Gurtschlitzen (114) am oberen Befestigungspunkt (216) zum Gürtel (206) am unteren Befestigungspunkt (302) erstrecken; wobei die unteren Befestigungspunkte (302) seitlich mit dem Gürtel (206) oder posterolateral mit dem Gürtel (206) verbunden sind.

8. Die physische Mensch-Roboter-Schnittstelle (100) nach Anspruch 1, wobei der obere Verbinder (204) des Stabilitätsgurtes mit dem zweiten Arm (112) verbunden ist und der untere Verbinder (304) des Stabilitätsgurtes mit dem zweiten Schultergurt (208) verbunden ist.

9. Die physische Mensch-Roboter-Schnittstelle (100) nach Anspruch 1, wobei der obere Verbinder (204) des Stabilitätsgurtes mit dem zweiten Arm (112) verbunden ist und der untere Verbinder (304) des Stabilitätsgurtes seitlich mit dem Gürtel (206) verbunden ist.

10. Die physische Mensch-Roboter-Schnittstelle (100) nach Anspruch 1, wobei der Stabilisierungsgurt (202) an der Seite, die dem zweiten Arm (112) entspricht, mit dem Gürtel (206) verbunden ist; wobei sich der Stabilisierungsgurt (202) vom mittleren aufrechten Abschnitt der Strebe (102) zu einer seitlichen Position des Gürtels (206) erstreckt, die der Seite des zweiten Arms (112) entspricht.

11. Die physische Mensch-Roboter-Schnittstelle (100) nach Anspruch 1, wobei sich der Stabilisierungsgurt (202) von einer Basis des zentralen aufrechten Abschnitts der Strebe (102) zu einer seitlichen Position des Gürtels (206) erstreckt, die der Seite des zweiten Arms (112) entspricht; wobei der Stabilisierungsgurt (202) konfiguriert ist, um sich im Wesentlichen horizontal um die Latissimus-dorsi-Muskeln eines Benutzers zu einem seitlich einer Hüfte eines Benutzers befindlichen Verankerungspunkt am Gürtel (206) erstreckt.

12. Die physische Mensch-Roboter-Schnittstelle (100) nach Anspruch 1, wobei der Stabilisierungsgurt (202) weiter einen Schnellverschluss (310) für den Stabilisierungsgurt umfasst.

## Revendications

1. Une interface physique homme-robot (100) destinée à être utilisée avec un exosquelette pour le haut du corps, ladite interface physique homme-robot comprenant : un montant rigide (102) définissant une ou plusieurs fentes pour sangle (114), ledit montant (102) étant adapté pour correspondre au dos d'un utilisateur ; une chaîne cinématique d'épaule (104) s'étendant depuis le montant [70] ; une ceinture (206) connectée au montant (102) ; deux bretelles (208, 210) configurées pour s'enrouler autour de l'épaule d'un utilisateur et s'étendant depuis la ou les fentes pour sangle (114) jusqu'à la ceinture (206) ;
dans laquelle le montant (102) est configuré en forme de T ayant un premier bras (110) et un second bras (112), le premier bras (110) et le second bras (112) s'étendant depuis une partie centrale verticale du montant (102), dans lequel la chaîne cinématique d'épaule se connecte au premier bras (110) d'un côté du plan sagittal de l'interface homme-robot,
dans laquelle les bretelles (208, 210) sont connectées respectivement au premier bras (110) et au second bras (112), dans lequel la ceinture (206) est connectée au montant (102) le long d'une base du montant (102),
dans laquelle une bretelle (208) s'ancre à la ceinture à une position située postérolatéralement ou latéralement le long de la ceinture du côté opposé du plan sagittal par rapport à la chaîne cinématique d'épaule (104),
dans laquelle l'interface comprend en outre une sangle de stabilité (202), **caractérisée en ce que** ladite sangle de stabilité (202) comprend une première extrémité fixée à un connecteur inférieur de sangle de stabilité (304) engageant la bretelle (208) qui est ancrée à la ceinture (206) du côté opposé du plan sagittal par rapport à la chaîne cinématique d'épaule (104) ou engageant la ceinture (206) à une position située antérolatéralement du même côté du plan sagittal que ladite bretelle (208) ancrée à la ceinture du côté opposé du plan sagittal par rapport à la chaîne cinématique d'épaule (104), et
la sangle de stabilité (202) ayant une seconde extrémité fixée à un connecteur supérieur de sangle de stabilité (204) engageant le montant depuis la partie centrale verticale du montant (102) ou depuis le second bras (112) du montant (102) de sorte que la sangle de stabilité s'étend depuis le montant dans une direction vers la ceinture ou la bretelle (208) à laquelle la sangle de stabilité est connectée à un élément de sécurité, afin de contrebalancer un moment de rotation causé par la chaîne cinématique d'épaule (104).

2. L'interface physique homme-robot (100) selon la revendication 1, dans laquelle la ou les bretelles (208, 210) ont un point d'attache supérieur (216) et un point d'attache inférieur (302), la ou les bretelles (208, 210) s'étendant depuis la ou les fentes pour sangle (114) au point d'attache supérieur (216) jusqu'à la ceinture (206) au point d'attache inférieur (302).

3. L'interface physique homme-robot (100) selon la revendication 1, dans laquelle le montant (102) comprend en outre un support lombaire (108).

4. L'interface physique homme-robot (100) selon la revendication 3, dans laquelle la ceinture (206) est connectée au support lombaire (108) du montant (102).

5. L'interface physique homme-robot (100) selon la revendication 4, dans laquelle la ceinture (206) comprend en outre un coussin lombaire (212).

6. L'interface physique homme-robot (100) selon la revendication 1, dans laquelle le premier bras (110) et le second bras (112) définissent la ou les fentes pour sangle (114).

7. L'interface physique homme-robot (100) selon la revendication 1, dans laquelle la ou les bretelles (208, 210) ont un point d'attache supérieur (216) et un point d'attache inférieur (302), la ou les bretelles (208, 210) s'étendant depuis la ou les fentes pour sangle (114) au point d'attache supérieur (216) jusqu'à la ceinture (206) au point d'attache inférieur (302) ; dans laquelle les points d'attache inférieurs (302) se connectent latéralement sur la ceinture (206), ou postérolatéralement sur la ceinture (206).

8. L'interface physique homme-robot (100) selon la revendication 1, dans laquelle le connecteur supérieur de sangle de stabilité (204) se connecte au second bras (112) et le connecteur inférieur de sangle de stabilité (304) se connecte à la seconde bretelle (208).

9. L'interface physique homme-robot (100) selon la revendication 1, dans laquelle le connecteur supérieur de sangle de stabilité (204) se connecte au second bras (112) et le connecteur inférieur de sangle de stabilité (304) se connecte latéralement sur la ceinture (206).

10. L'interface physique homme-robot (100) selon la revendication 1, dans laquelle la sangle de stabilité (202) se connecte à la ceinture (206) du côté correspondant au second bras (112) ; dans laquelle la sangle de stabilité (202) s'étend depuis la partie centrale verticale du montant (102) jusqu'à une position latérale de la ceinture (206) correspondant au côté du second bras (112).

11. L'interface physique homme-robot (100) selon la revendication 1, dans laquelle la sangle de stabilité (202) s'étend depuis une base de la partie centrale verticale du montant (102) jusqu'à une position latérale de la ceinture (206) correspondant au côté du second bras (112) ; dans laquelle la sangle de stabilité (202) est configurée pour s'étendre de manière substantiellement horizontale autour des muscles grand dorsal d'un utilisateur jusqu'à un point d'ancrage sur la ceinture (206) situé latéralement à la hanche d'un utilisateur.

12. L'interface physique homme-robot (100) selon la revendication 1, dans laquelle la sangle de stabilité (202) comprend en outre un dispositif de libération rapide de sangle de stabilité (310).
